(19) Europäisches Patentamt
European Patent Office
Office européen des brevets

(11) EP 3 498 745 A1

(12) **EUROPÄISCHE PATENTANMELDUNG**

(43) Veröffentlichungstag:
**19.06.2019 Patentblatt 2019/25**

(21) Anmeldenummer: **17207933.7**

(22) Anmeldetag: **18.12.2017**

(51) Int Cl.:
*C08G 18/50* (2006.01) *C08G 18/66* (2006.01)
*C08G 18/76* (2006.01) *C08J 9/14* (2006.01)
*C08G 18/09* (2006.01) *C08G 18/18* (2006.01)
*C08G 18/22* (2006.01) *C08G 18/38* (2006.01)
*C08G 18/40* (2006.01) *C08G 18/42* (2006.01)
*C07C 269/04* (2006.01) *C07C 271/20* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **FLAMMGESCHÜTZTE POLYURETHAN-HARTSCHAUMSTOFFE**

(57) Die vorliegende Erfindung betrifft flammgeschützte Polyurethan-Hartschaumstoffe bzw. Polyurethan/Polyisocyanurat-Hartschaumstoffe (im Folgenden einzeln oder gemeinsam auch als "PUR-/PIR-Hartschaumstoffe" bezeichnet) mit Tri-Urethan-Triolen (im Folgenden auch als "TUT" bezeichnet), sowie Mischungen enthaltend ein Tri-Urethan-Triol und eine Komponente ausgewählt aus der Gruppe bestehend aus Polyetherpolyol, Polyesterpolyol, Polyethercarbonatpolyol und Polyetheresterpolyol und Verfahren zur Herstellung von PUR-/PIR-Hartschaumstoffen, enthaltend
**A1** eine Isocyanatreaktive Komponente
**A2** Flammschutzmittel
**A3** Treibmittel
**A4** Katalysator
**A5** gegebenenfalls Hilfs- und Zusatzstoffe
**B** eine organische Polyisocyanatkomponente
dadurch gekennzeichnet, dass
die Komponente **A1** ein Tri-Urethan-Triol **A1.1** und eine Verbindung **A1.2** ausgewählt aus der Gruppe bestehend aus Polyetherpolyol, Polyesterpolyol, Polyethercarbonatpolyol und Polyetheresterpolyol enthält.

EP 3 498 745 A1

## Beschreibung

Die vorliegende Erfindung betrifft flammgeschützte Polyurethan-Hartschaumstoffe bzw. Polyurethan/Polyisocyanurat-Hartschaumstoffe (im Folgenden einzeln oder gemeinsam auch als "PUR-/PIR-Hartschaumstoffe" bezeichnet) mit Tri-Urethan-Triolen (im Folgenden auch als "TUT" bezeichnet), sowie Verfahren zu ihrer Herstellung und die Verwendung von Tri-Urethan-Triolen in Systemen zur Herstellung von PUR-/PIR-Hartschaumstoffen und Mischungen enthaltend ein Tri-Urethan-Triol und eine Verbindung ausgewählt aus der Gruppe bestehend aus Polyetherpolyol, Polyesterpolyol, Polyethercarbonatpolyol und Polyetheresterpolyol.

Die Herstellung von Polyethercarbonatpolyolen durch katalytische Umsetzung von Alkylenoxiden (Epoxiden) und Kohlendioxid in Anwesenheit von H-funktionellen Startersubstanzen ("Starter") wird seit mehr als 40 Jahren intensiv untersucht (z. B. Inoue et al., Copolymerization of Carbon Dioxide and Epoxide with Organometallic Compounds; Die Makromolekulare Chemie 130, 210-220, 1969). Diese Reaktion ist in Schema (I) schematisch dargestellt, wobei R für einen organischen Rest wie Alkyl, Alkylaryl oder Aryl steht, der jeweils auch Heteroatome wie beispielsweise O, S, Si usw. enthalten kann, und wobei e, f und g für eine ganzzahlige Zahl stehen, und wobei das hier im Schema (I) gezeigte Produkt für das Polyethercarbonatpolyol lediglich so verstanden werden soll, dass sich Blöcke mit der gezeigten Struktur im erhaltenen Polyethercarbonatpolyol prinzipiell wiederfinden können, die Reihenfolge, Anzahl und Länge der Blöcke sowie die OH-Funktionalität des Starters aber variieren kann und nicht auf das in Schema (I) gezeigte Polyethercarbonatpolyol beschränkt ist. Diese Reaktion (siehe Schema (I)) ist ökologisch sehr vorteilhaft, da diese Reaktion die Umsetzung eines Treibhausgases wie $CO_2$ zu einem Polymer darstellt und so eine entsprechende Menge Alkylenoxid ersetzt, d.h. einspart. Als Nebenprodukt, entsteht das in Schema (I) gezeigte cyclische Carbonat (beispielsweise für R = $CH_3$ Propylencarbonat, im Folgenden auch als cPC bezeichnet, oder für R = H Ethylencarbonat, im Folgenden auch als cEC bezeichnet).

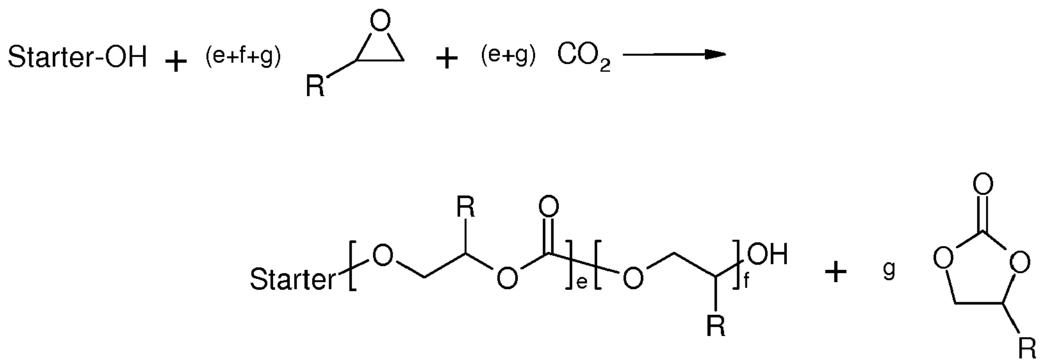

(I)

Für die Herstellung derartiger Polyethercarbonatpolyole haben sich Doppelmetallcyanid (DMC)-Katalysatoren als besonders vorteilhaft erwiesen.

In EP 3 098 251 A1 wird ein Verfahren zur Herstellung von Diurethandiolen aus cyclischen Carbonaten und Diaminen offenbart. Die erhaltenen Diurethandiole werden als H-funktionelle Starterverbindung zur Herstellung von Polyetherpolyolen eingesetzt. Des Weiteren wird ein Verfahren zur Herstellung von Polyurethan-Weichschaumstoffen aus den erhaltenen Polyetherpolyolen offenbart. Es werden jedoch weder Tri-Urethan-Triole offenbart, noch der Einsatz von Diurethandiolen als Isocyanatreaktive Komponente offenbart.

Die der vorliegenden Erfindung zu Grunde liegende Aufgabe bestand deshalb darin, das in der Herstellung von Polyethercarbonatpolyolen als Nebenprodukt anfallende cyclische Carbonat für die Herstellung von flammgeschützten Polyurethan-Hartschaumstoffen stofflich zu verwerten.

Eine weitere Aufgabe bestand darin, den Flammschutz von Polyurethan-Hartschaumstoffen zu verbessern und den Anteil von halogenierten Flammschutzmitteln in der Herstellung von Polyurethan-Hartschaumstoffen zu verringern.

Diese Aufgabe wird erfindungsgemäß gelöst durch ein Verfahren zur Herstellung von PUR-/PIR-Hartschaumstoffen, enthaltend

**A1** eine Isocyanatreaktive Komponente

**A2** Flammschutzmittel

**A3** Treibmittel

**A4** Katalysator

**A5** gegebenenfalls Hilfs- und Zusatzstoffe

**B** eine organische Polyisocyanatkomponente

dadurch gekennzeichnet, dass
die Isocyanatreaktive Komponente **A1** ein Tri-Urethan-Triol **A1.1** und eine Verbindung **A1.2** ausgewählt aus der Gruppe bestehend aus Polyetherpolyol, Polyesterpolyol, Polyethercarbonatpolyol und Polyetheresterpolyol enthält.

Überraschend wurde gefunden, dass eine Mischung enthaltend ein Tri-Urethan-Triol **A1.1** und eine Verbindung **A1.2,** dadurch gekennzeichnet dass, die Verbindung **A1.2** ausgewählt ist aus der Gruppe bestehend aus Polyetherpolyol, Polyesterpolyol, Polyethercarbonatpolyol und Polyetheresterpolyol, in einem Verfahren zur Herstellung von PUR-/PIR-Hartschaumstoffen den Flammschutz der erhaltenen PUR-/PIR-Hartschaumstoffe erhöht.

Die Verwendung des Worts *ein* im Zusammenhang mit zählbaren Größen ist hierbei und im Folgenden nur dann als Zahlwort zu verstehen, wenn dies aus dem Zusammenhang hervorgeht (bspw. durch die Formulierung *"genau ein"*). Ansonsten umfassen Ausdrücke wie "ein Alkylenoxid", "ein Tri-Urethan-Triol **A1.1**" etc. immer auch solche Ausführungsformen, in denen zwei oder mehr Alkylenoxide, zwei oder mehr Tri-Urethan-Triole **A1.1,** etc. eingesetzt werden.

Unter Äquivalentmolmasse ist die durch die Zahl der aktiven Wasserstoffatome geteilte Gesamtmasse des aktive Wasserstoffatome enthaltenden Materials zu verstehen. Im Falle von hydroxygruppenhaltigen Materialien steht sie in folgender Beziehung zur OH-Zahl:

$$\text{Äquivalentmolmasse} = 56100 / \text{OH-Zahl [mg KOH/g]}$$

Die Äquivalentmolmasse steht mit der Molmasse in folgender Beziehung:

$$\text{Molmasse} = \text{Äquivalentmolmasse} * \text{Funktionalität}$$

Mit Funktionalität wird die Anzahl an aktiven Wasserstoffatomen pro Einzelmolekül bezeichnet. Im Rahmen der vorliegenden Erfindung handelt es sich bei den Begriffen Äquivalentmolmasse, Molmasse und Funktionalität um zahlenmittlere Größen.

Die OH-Zahl (auch: Hydroxylzahl) gibt im Falle eines einzelnen zugesetzten Polyols dessen OH-Zahl an. Angaben der OH-Zahl für Mischungen beziehen sich auf die zahlenmittlere OH-Zahl der Mischung, berechnet aus den OH-Zahlen der einzelnen Komponenten in ihren jeweiligen molaren Anteilen. Die OH-Zahl gibt die Menge an Kaliumhydroxid in Milligramm an, welche der bei einer Acetylierung von einem Gramm Substanz gebundenen Menge Essigsäure gleichwertig ist. Die OH-Zahl wird im Rahmen der vorliegenden Erfindung nach der Norm DIN 53240-1 (Juni 2013) bestimmt.

Nachfolgend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

Erfindungsgemäß enthält die Isocyanat-reaktive Komponente **A1** ein Tri-Urethan-Triol **A1.1** und eine Verbindung **A1.2.** Das erfindungsgemäß eingesetzte Tri-Urethan-Triol **A1.1** ist erhältlich durch Umsetzung von cyclischen Carbonaten mit Verbindungen die mindestens drei Aminogruppen enthalten, wobei mindestens zwei der Aminogruppen primäre Aminogruppen sind. Beispielsweise sind die Tri-Urethan-Triole **A1.1** erhältlich durch Umsetzung von Propylencarbonat und/oder Ethylencarbonat mit Verbindungen die drei Aminogruppen enthalten, wobei mindestens zwei der Aminogruppen primäre Aminogruppen sind.

Bevorzugt sind Tri-Urethan-Triole **A1.1** gemäß der Formel (II),

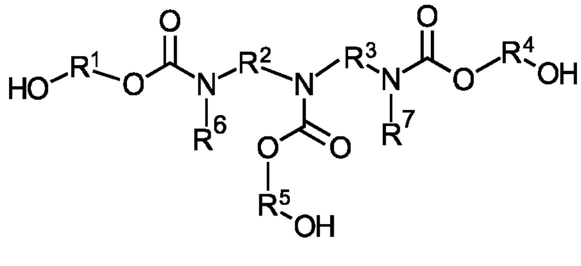

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^2$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^3$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei $R^1$ bis $R^7$ identisch oder voneinander verschieden sein können.

Die bevorzugten Tri-Urethan-Triole **A1.1** gemäß der Formel (II) werden erhalten durch Umsetzung von cyclischen Carbonaten mit Triaminen gemäß Formel (III),

$$\mathbf{HN(R^6)\text{-}R^2\text{-}N(R^8)\text{-}R^3\text{-}NH(R^7)} \qquad \text{(III)}$$

wobei $R^2$, $R^3$, $R^6$, $R^7$ und $R^8$ die oben genannte Bedeutung haben, wobei $R^2$, $R^3$, $R^6$, $R^7$ und $R^8$ identisch oder voneinander verschieden sein können.

Als cyclische Carbonate werden bevorzugt Propylencarbonat und/oder Ethylencarbonat eingesetzt.

Beispielsweise werden die Tri-Urethan-Triole **A1.1** gemäß der Formel (II) durch Umsetzung von Propylencarbonat und/oder Ethylencarbonat mit Triaminen gemäß Formel (III) erhalten.

Besonders bevorzugt werden die Tri-Urethan-Triole **A1.1** der Formel (II) durch Umsetzung von Propylencarbonat und/oder Ethylencarbonat mit mindestens einer Verbindung ausgewählt aus der Gruppe bestehend aus Diethylentriamin, 1,5,10-Triazadecan, 1,12-Diamino-4,9-diazadodekan, Dipropylentriamin, N-(2-Aminoethyl)-1,3-propandiamin und N,N'-Bis(3-aminopropyl)ethylendiamin erhalten.

Die Umsetzung der cyclischen Carbonate mit Verbindungen, die mindestens drei Aminogruppen enthalten, wobei mindestens zwei der Aminogruppen primäre Aminogruppen sind, erfolgt bevorzugt bei 50 bis 150 °C, besonders bevorzugt bei 80 bis 120 °C. Die Reaktionszeit liegt bevorzugt bei 4 bis 40 h, besonders bevorzugt bei 6 bis 30 h. Die Reaktion kann dabei in Anwesenheit eines Katalysators für die Aminolyse, wie z. B. 1,8-Diazabicyclo(5.4.0)undec-7-en, stattfinden.

In einer besonders vorteilhaften Ausführungsform wird das cyclische Carbonat mindestens äquimolar bezogen auf cyclisches Carbonat und Aminogruppen eingesetzt. Bevorzugt beträgt das molare Verhältnis von cyclischem Carbonat zu den Aminogruppen der Verbindungen die mindestens drei Aminogruppen enthalten, wobei mindestens zwei der Aminogruppen primäre Aminogruppen sind , 1,0 bis 4,0, besonders bevorzugt von 1,4 bis 3, ganz besonders bevorzugt von 2,0 bis 2,6.

Das überschüssige cyclische Carbonat kann direkt nach der Synthese des Tri-Urethan-Triols **A1.1** durch z.B. Dünnschichtverdampfung entfernt werden.

Es können jedoch auch molare Verhältnisse von cyclischem Carbonat zu den Aminogruppen der Verbindungen

die mindestens drei Aminogruppen enthalten, wobei mindestens zwei der Aminogruppen primäre Aminogruppen sind, von unter 1,00 gewählt werden.

Der Anteil des Tri-Urethan-Triols **A1.1** in dem Verfahren kann 0,1 bis 35,0 Gew.-%, bevorzugt 3,0 bis 25,0 Gew.-%, besonders bevorzugt 5,0 bis 15,0 Gew.-% bezogen auf die Summe der Massen der Komponenten **A1.1** und **A1.2** betragen.

Es können in der Isocyanat-reaktiven Verbindung **A1** ein oder mehrere Tri-Urethan-Triole **A1.1** enthalten sein.

Neben dem Tri-Urethan-Triol **A1.1** werden im erfindungsgemäßen Verfahren mindestens eine Verbindung **A1.2** ausgewählt aus der Gruppe bestehend aus Polyetherpolyolen, Polyesterpolyolen, Polyetheresterpolyolen, Polycarbonatpolyolen und Polyethercarbonatpolyolen eingesetzt. Bevorzugt sind Polyesterpolyole und/oder Polyetherpolyole und ganz besonders bevorzugt Polyesterpolyole. Die Verbindung **A1.2** kann bevorzugt eine Hydroxylzahl zwischen 100 bis 550 mg KOH/g, insbesondere 100 bis 450 mg KOH/g und besonders bevorzugt 100 bis 350 mg KOH/g aufweisen. Vorzugsweise weist die einzelne Polyolkomponente ein zahlenmittleres Molekulargewicht von 120 g/mol bis 6000 g/mol, insbesondere 400 g/mol bis 2000 g/mol und besonders bevorzugt 500 g/mol bis 1000 g/mol auf. Die OH-Funktionalität der Verbindung **A1.2** kann 1,5 bis 4,0, bevorzugt 1,8 bis 3,0 und besonders bevorzugt 1,9 bis 2,5 betragen.

Die Polyesterpolyole der Verbindung **A1.2** können beispielsweise Polykondensate aus mehrwertigen Alkoholen, vorzugsweise Diolen, mit 2 bis 12 Kohlenstoffatomen, vorzugsweise mit 2 bis 6 Kohlenstoffatomen, und Polycarbonsäuren, wie z.B. Di-, Tri- oder sogar Tetracarbonsäuren, oder Hydroxycarbonsäuren oder Lactonen sein, bevorzugt werden aromatische Dicarbonsäuren oder Gemische aus aromatischen und aliphatischen Dicarbonsäuren verwendet. Es können aber auch nur eine oder mehrere aliphatische Polycarbonsäuren verwendet werden. Anstelle der freien Polycarbonsäuren können auch die entsprechenden Polycarbonsäureanhydride oder entsprechende Polycarbonsäureester von niederen Alkoholen zur Herstellung der Polyester verwendet werden, wobei bevorzugt Phthalsäureanhydrid eingesetzt wird.

Als Carbonsäuren kommen insbesondere in Betracht: Bernsteinsäure, Glutarsäure, Adipinsäure, Korksäure, Azelainsäure, Sebazinsäure, Decandicarbonsäure, Tetrahydrophthalsäure, Hexahydrophthalsäure, Cyclohexandicarbonsäure, Tetrachlorphthalsäure, Itaconsäure, Malonsäure, 2-Methylbernsteinsäure, 3,3-Diethylglutarsäure, 2,2-Dimethylbernsteinsäure, Dodekandisäure, Endomethylentetrahydrophthalsäure, Dimerfettsäure, Trimerfettsäure, Zitronensäure, Trimellithsäure, Benzoesäure, Trimellitsäure, Maleinsäure, Fumarsäure, Phthalsäure, Isophthalsäure und Terephthalsäure. Verwendet werden können ebenso Derivate dieser Carbonsäuren, wie beispielsweise Dimethylterephthalat. Die Carbonsäuren können dabei sowohl einzeln als auch im Gemisch verwendet werden. Als Carbonsäuren werden bevorzugt Terephthalsäure, Isophthalsäure, Adipinsäure, Sebacinsäure, Glutarsäure und/oder Bernsteinsäure, besonders bevorzugt Adipinsäure, Glutarsäure und/oder Bernsteinsäure und deren Gemische verwendet.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind unter anderem Caprolacton, Butyrolacton und Homologe.

Zur Herstellung der Polyesterpolyole kommen insbesondere auch biobasierte Ausgangsstoffe und/oder deren Derivate in Frage, wie z. B. Rizinusöl, Polyhydroxyfettsäuren, Ricinolsäure, Hydroxylmodifizierte Öle, Weintraubenkernöl, schwarzem Kümmelöl, Kürbiskernöl, Borretschsamenöl, Sojabohnenöl, Weizensamenöl, Rapsöl, Sonnenblumenkernöl, Erdnussöl, Aprikosenkernöl, Pistazienöl, Mandelöl, Olivenöl, Macadamianussöl, Avocadoöl, Sanddornöl, Sesamöl, Hanföl, Haselnussöl, Primelöl, Wildrosenöl, Distelöl, Walnussöl, Fettsäuren, hydroxylmodifizierte und epoxidierte Fettsäuren und Fettsäureester, beispielsweise basierend auf Myristoleinsäure, Palmitoleinsäure, Ölsäure, Vaccensäure, Petroselinsäure, Gadoleinsäure, Erukasäure, Nervonsäure, Linolsäure, alpha- und gamma-Linolensäure, Stearidonsäure, Arachidonsäure, Timnodonsäure, Clupanodonsäure und Cervonsäure. Insbesondere bevorzugt sind Ester der Rizinolsäure mit mehrfunktionellen Alkoholen, z.B. Glycerin. Bevorzugt ist auch die Verwendung von Mischungen solcher biobasierten Säuren mit anderen Carbonsäuren, z.B. Phthalsäuren.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester. Vorzugsweise verwendet werden Ethylenglykol, Diethylenglykol, 1,4-Butandiol, 1,5-Pentandiol, 1,6-Hexandiol oder Mischungen aus mindestens zwei der genannten Diole, insbesondere Mischungen aus 1,4-Butandiol, 1,5-Pentandiol und 1,6-Hexandiol. Ganz besonders bevorzugt werden Ethylenglykol und Diethylenglykol verwendet.

Daneben können auch Polyole wie Trimethylolpropan, Glycerin, Erythrit, Pentaerythrit, Trimethylolbenzol oder Trishydroxyethylisocyanurat eingesetzt werden, wobei Glycerin und Trimethylolpropan bevorzugt sind.

Es können zusätzlich auch einwertige Alkanole mit verwendet werden.

Erfindungsgemäß eingesetzte Polyetherpolyole werden nach dem Fachmann bekannten Herstellungsmethoden erhalten, wie beispielsweise durch anionische Polymerisation von einem oder mehreren Alkylenoxiden mit 2 bis 4 Kohlenstoffatomen mit Alkalihydroxiden, wie Natrium- oder Kaliumhydroxid, Alkalialkoholaten, wie Natriummethylat, Natrium- oder Kaliumethylat oder Kaliumisopropylat, oder aminischen Alkoxylierungs-Katalysatoren, wie Dimethylethanolamin (DMEOA), Imidazol und/oder Imidazolderivate, unter Verwendung mindestens eines Startermoleküls, das 2 bis 8, vorzugsweise 2 bis 6 reaktive Wasserstoffatome gebunden enthält.

Geeignete Alkylenoxide sind beispielsweise 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Bevorzugte Alkylenoxide sind Propylenoxid und Ethylenoxid.

Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N- und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,3-, 2,4- und 2,6-Toluylendiamin und 2,2'-, 2,4'- und 4,4'-Diaminodiphenylmethan.

Vorzugsweise verwendet werden zwei oder mehrwertige Alkohole, wie Ethandiol, 1,2- und 1,3-Propandiol, Diethylenglykol, Dipropylenglykol, 1,4-Butandiol, 1,6-Hexandiol, Triethanolamin, Bisphenole, Glycerin, Trimethylolpropan, Pentaerythrit, Sorbit und Saccharose.

Verwendbare Polycarbonatpolyole sind Hydroxylgruppen aufweisende Polycarbonate, zum Beispiel Polycarbonatdiole. Diese entstehen in der Reaktion von Kohlensäurederivaten, wie Diphenylcarbonat, Dimethylcarbonat oder Phosgen, mit Polyolen, bevorzugt Diolen.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenole und lactonmodifizierte Diole der vorstehend genannten Art.

Statt oder zusätzlich zu reinen Polycarbonatdiolen können auch Polyether-Polycarbonatdiole eingesetzt werden, welche beispielsweise durch Copolymerisation von Alkylenoxiden, wie zum Beispiel Propylenoxid, mit $CO_2$ erhältlich sind.

Verwendbare Polyetheresterpolyole sind solche Verbindungen, die Ethergruppen, Estergruppen und OH-Gruppen enthalten. Organische Dicarbonsäuren mit bis zu 12 Kohlenstoffatomen sind zur Herstellung der Polyetheresterpolyole geeignet, vorzugsweise aliphatische Dicarbonsäuren mit 4 bis 6 Kohlenstoffatomen oder aromatische Dicarbonsäuren, die einzeln oder im Gemisch verwendet werden. Beispielhaft seien Korksäure, Azelainsäure, Decandicarbonsäure, Maleinsäure, Malonsäure, Phthalsäure, Pimelinsäure und Sebacinsäure sowie insbesondere Glutarsäure, Fumarsäure, Bernsteinsäure, Adipinsäure, Phthalsäure, Terephthalsäure und Isophthalsäure genannt. Neben organischen Dicarbonsäuren können auch Derivate dieser Säuren, beispielsweise deren Anhydride sowie deren Ester und Halbester mit niedermolekularen, monofunktionellen Alkoholen mit 1 bis 4 Kohlenstoffatomen eingesetzt werden. Der anteilige Einsatz der oben genannten biobasierten Ausgangsstoffe, insbesondere von Fettsäuren bzw. Fettsäurederivaten (Ölsäure, Sojaöl etc.) ist ebenfalls möglich und kann Vorteile aufweisen, z.B. im Hinblick auf Lagerstabilität der Polyolformulierung, Dimensionsstabilität, Brandverhalten und Druckfestigkeit der Schäume.

Als weitere Komponente zur Herstellung der Polyetheresterpolyole werden Polyetherpolyole eingesetzt, die man durch Alkoxylieren von Startermolekülen wie mehrwertigen Alkoholen erhält. Die Startermoleküle sind mindestens difunktionell, können aber gegebenenfalls auch Anteile höherfunktioneller, insbesondere trifunktioneller, Startermoleküle enthalten.

Startermoleküle sind zum Beispiel Diole wie 1,2-Ethandiol, 1,3-Propandiol, 1,2-Propandiol, 1,4-Butandiol, 1,5-Pentendiol, 1,5-Pentandiol, Neopentylglykol, 1,6- Hexandiol, 1,7-Heptandiol, 1,8-Octandiol, 1,10-Decandiol, 2-Methyl-1,3-propandiol, 2,2-Dimethyl-1,3-propandiol, 3-Methyl-1,5-pentandiol, 2-Butyl-2-ethyl-1,3-propandiol, 2-Buten-1,4-diol und 2-Butin-1,4-diol, Etherdiole wie Diethylenglykol, Triethylenglykol, Tetraethylenglykol, Dibutylenglykol, Tributylenglykol, Tetrabutylenglykol, Dihexylenglykol, Trihexylenglykol, Tetrahexylenglykol und Oligomerengemische von Alkylenglykolen, wie Diethylenglykol. Auch Startermoleküle mit von OH verschiedenen Funktionalitäten können alleine oder in Mischung eingesetzt werden.

Neben den Diolen können als Startermoleküle für die Herstellung der Polyether auch Verbindungen mit mehr als 2 Zerewitinoff-aktiven Wasserstoffen, besonders mit zahlenmittleren Funktionalitäten von 3 bis 8, insbesondere von 3 bis 6 mitverwendet werden, zum Beispiel 1,1,1-Trimethylolpropan, Triethanolamin, Glycerin, Sorbitan und Pentaerythrit sowie auf Triolen oder Tetraolen gestartete Polyethylenoxidpolyole.

Polyetheresterpolyole können auch durch die Alkoxylierung, insbesondere durch Ethoxylierung und/oder Propoxylierung, von Reaktionsprodukten, die durch die Umsetzung von organischen Dicarbonsäuren und deren Derivaten sowie Komponenten mit Zerewitinoff-aktiven Wasserstoffen, insbesondere Diolen und Polyolen, erhalten werden, hergestellt werden. Als Derivate dieser Säuren können beispielsweise deren Anhydride eingesetzt werden, wie zum Beispiel Phthalsäureanhydrid.

Herstellungsverfahren der Polyole sind beispielsweise von Ionescu in "Chemistry and Technology of Polyols for Polyurethanes", Rapra Technology Limited, Shawbury 2005, S.55 ff. (Kap. 4: Oligo-Polyols for Elastic Polyurethanes), S. 263 ff. (Kap. 8: Polyester Polyols for Elastic Polyurethanes) und insbesondere auf S.321 ff. (Kap. 13: Polyether Polyols

for Rigid Polyurethane Foams) und S.419 ff. (Kap. 16: Polyester Polyols for Rigid Polyurethane Foams) beschrieben worden. Es ist auch möglich, Polyester- und Polyetherpolyole über Glykolyse von geeigneten Polymer-Rezyklaten zu gewinnen. Geeignete Polyether-Polycarbonatpolyole und ihre Herstellung werden beispielsweise in der EP 2 910 585 A1, [0024]-[0041], beschrieben. Beispiele zu Polycarbonatpolyolen und ihre Herstellung finden sich unter anderem in der EP 1 359 177 A1. Die Herstellung geeigneter Polyetheresterpolyole ist unter anderem in der WO 2010/043624 A und in der EP 1 923 417 A beschrieben.

Die Verbindung **A1.2** kann eine oder mehrere der oben genannten Verbindungen enthalten.

Weiterhin können in der Isocyanat-reaktiven Komponente **A1** niedermolekulare Isocyanat-reaktive Verbindungen **A1.3** enthalten sein, insbesondere können di- oder trifunktionelle Amine und Alkohole, besonders bevorzugt Diole und/oder Triole mit Molmassen $M_n$ kleiner als 400 g/mol, vorzugsweise von 60 bis 300 g/mol, zum Einsatz kommen, z.B. Triethanolamin, Diethylenglykol, Ethylenglykol und Glycerin. Sofern zur Herstellung der Polyurethan-Hartschaumstoffe solche niedermolekularen Isocyanat-reaktiven Verbindungen Anwendung finden, z.B. in der Funktion als Kettenverlängerungsmittel und/oder Vernetzungsmittel kommen diese zweckmäßigerweise in einer Menge von bis zu 5 Gew.-%, bezogen auf das Gesamtgewicht der Isocyanatreaktiven Komponente **A1**, zum Einsatz.

Neben den oben beschriebenen Polyolen und Isocyanatreaktiven Verbindungen können in der Isocyanatreaktiven Komponente **A1** weitere Isocyanat-reaktive Verbindungen **A1.4** enthalten sein, beispielsweise Graft-Polyole, Polyamine, Polyaminoalkohole und Polythiole. Selbstverständlich umfassen die beschriebenen Isocyanat-reaktiven Komponenten auch solche Verbindungen mit gemischten Funktionalitäten.

Die Isocyanat-reaktive Komponente **A1** kann eine oder mehrere der oben genannten Verbindungen enthalten. In einer bevorzugten Ausführungsform enthält die Isocyanatreaktive Komponente **A1** nur Tri-Urethan-Triol **A1.1** und Verbindung **A1.2.**

Als Flammschutzmittel **A2** können Verbindungen wie beispielsweise Phosphate oder Phosphonate, wie z. B. Diethylethylphosphonat (DEEP), Triethylphosphat (TEP), Triphenylphosphat (TPP), Trikresylphosphat, Diphenylkresylphosphat (DPK), Dimethylmethylphosphonat (DMMP), Diethanolaminomethylphosphonsäurediethylester, 9,10-Dihydro-9-oxa-10-phosphorylphenanthrene-10-oxid (DOPO) und Dimethylpropylphosphonat (DMPP), eingesetzt werden. Weitere geeignete Flammschutzmittel **A2** sind beispielsweise bromierte Ester, bromierte Ether (Ixol) oder bromierte Alkohole wie Dibromneopentylalkohol, Tribromneopentylalkohol, Tetrabromphthalat-Diol, sowie chlorierte Phosphate wie Tris(2-chlorethyl)phosphat, Tris-(2-chlorpropyl)phosphat (TCPP), Tris(1,3-dichlorpropyl)phosphat, Tris-(2,3-dibrompropyl)phosphat, Tetrakis-(2-chlorethyl)-ethylendiphosphat sowie handelsübliche halogenhaltige Flammschutzpolyole. Der Anteil des Flammschutzmittels **A2** kann 2,0 Gew.-% bis 30,0 Gew.-%, bevorzugt 4,0 Gew.-% bis 25,0 Gew.-%, besonders bevorzugt 8,0 Gew.-% bis 20,0 Gew.-%, bezogen auf die Summe der Massen der Komponenten **A1** bis **A5,** betragen.

Als Treibmittel **A3** kann physikalisches Treibmittel eingesetzt werden, wie beispielsweise niedrig siedende organische Verbindungen wie z. B. Kohlenwasserstoffe, halogenierte Kohlenwasserstoffe, Ether, Ketone, Carbonsäureester oder Kohlensäureester. Geeignet sind insbesondere organische Verbindungen, welche gegenüber der Isocyanatkomponente **B** inert sind und Siedepunkte unter 100 °C, vorzugsweise unter 50 °C bei Atmosphärendruck aufweisen. Diese Siedepunkte haben den Vorteil, dass die organischen Verbindungen unter dem Einfluss der exothermen Polyadditionsreaktion verdampfen. Beispiele solcher, vorzugsweise verwendeten organischen Verbindungen sind Alkane, wie Heptan, Hexan, n- und iso-Pentan, vorzugsweise technische Gemische aus n- und iso-Pentanen, n- und iso-Butan und Propan, Cycloalkane, wie z.B. Cyclopentan und/oder Cyclohexan, Ether, wie z. B. Furan, Dimethylether und Diethylether, Ketone, wie z. B. Aceton und Methylethylketon, Carbonsäurealkylester, wie z. B. Methylformiat, Dimethyloxalat und Ethylacetat und halogenierte Kohlenwasserstoffe, wie z. B. Methylenchlorid, Dichlormonofluormethan, Difluormethan, Trifluormethan, Difluorethan, Tetrafluorethan, Chlordifluorethane, 1,1-Dichlor-2,2,2-trifluorethan, 2,2-Dichlor-2-fluorethan und Heptafluorpropan. Auch bevorzugt ist der Einsatz von (hydro)fluorierten Olefinen, wie z. B. HFO 1233zd(E) (Trans-1-chlor-3,3,3-trifluor-1-propen) oder HFO 1336mzz(Z) (cis-1,1,1,4,4,4-Hexafluor-2-buten) oder Additive wie FA 188 von 3M (1,1,1,2,3,4,5,5,5-Nonafluor-4-(trifluormethyl)pent-2-en). Auch Gemische zweier oder mehrerer der genannten organischen Verbindungen können verwendet werden. Die organischen Verbindungen können dabei auch in Form einer Emulsion aus kleinen Tröpfchen eingesetzt werden.

Als Treibmittel **A3** kann auch chemisches Treibmittel, wie beispielsweise Wasser, Carbonsäure und deren Gemische, verwendet werden. Diese reagieren mit Isocyanatgruppen unter Bildung des Treibgases, wie beispielsweise im Falle von Wasser entsteht dabei Kohlendioxid und im Falle von z. B. Ameisensäure entsteht dabei Kohlendioxid und Kohlenstoffmonoxid. Als Carbonsäure wird bevorzugt mindestens eine Verbindung, ausgewählt aus der Gruppe bestehend aus Ameisensäure, Essigsäure, Oxalsäure und Ricinolsäure, eingesetzt. Als chemisches Treibmittel wird besonders bevorzugt Wasser eingesetzt.

Vorzugsweise werden keine halogenierten Kohlenwasserstoffe als Treibmittel verwendet.

Als Treibmittel **A3** wird mindestens eine Verbindung, ausgewählt aus der Gruppe bestehend aus physikalischen und chemischen Treibmittel, eingesetzt. Bevorzugt wird nur physikalisches Treibmittel eingesetzt.

Als Katalysator **A4** zur Herstellung der PUR-/PIR-Hartschaumstoffe werden Verbindungen verwendet, welche

die Reaktion der reaktiven Wasserstoffatome, insbesondere Hydroxylgruppen enthaltenden Verbindungen mit der Isocyanatkomponente **B** beschleunigen, wie z. B. tertiäre Amine oder Metallsalze. Die Katalysatorkomponenten können dem Reaktionsgemisch zudosiert oder auch ganz oder teilweise in der Isocyanatreaktiven Komponente **A1** vorgelegt werden.

Verwendet werden beispielsweise tertiäre Amine, wie Triethylamin, Tributylamin, Dimethylbenzylamin, Dicyclohexylmethylamin, Dimethylcyclohexylamin, N, N ,N', N'-Tetramethyldiaminodiethylether, Bis-(dimethylaminopropyl)-harnstoff, N-Methyl- bzw. N-Ethylmorpholin, N-Cyclohexylmorpholin, N,N,N',N'-Tetramethylethylendiamin, N,N,N,N-Tetramethylbutandiamin, N, N, N, N-Tetramethylhexandiamin-1,6, Pentamethyldiethylentriamin, Bis[2-(dimethylamino)ethyl]ether, Dimethylpiperazin, N-Dimethylaminoethylpiperidin, 1,2-Dimethylimidazol, 1-Azabicyclo-(3,3,0)-octan, 1,4-Diaza-bi-cyclo-(2,2,2)-octan (Dabco) und Alkanolaminverbindungen, wie Triethanolamin, Triisopropanolamin, N-Methyl- und N-Ethyldiethanolamin, Dimethylaminoethanol, 2-(N,N-Dimethylaminoethoxy)ethanol, N,N',N"-Tris-(dialkylaminoalkyl)hexahydrotriazin, z.B. N,N',N"-Tris-(dimethylaminopropyl)hexahydrotriazin und Triethylendiamin.

Es können auch Metallsalze, wie z. B. Alkali- oder Übergangsmetallsalze eingesetzt werden. Als Übergangsmetallsalze werden beispielsweise Zink-, Wismut-, Eisen-, Blei- oder bevorzugt Zinnsalze eingesetzt. Beispiele für eingesetzte Übergangsmetallsalze sind Eisen(II)-chlorid, Zinkchlorid, Bleioctoat, Zinndioctoat, Zinndiethylhexoat und Dibutylzinndilaurat. Besonders bevorzugt ist das Übergangsmetallsalz ausgewählt aus mindestens einer Verbindung aus der Gruppe bestehend aus Zinndioctoat, Zinndiethylhexoat und Dibutylzinndilaurat. Beispiele für Alkalimetallsalze sind Alkalialkoholate, wie z. B. Natriummethylat und Kaliumisopropylat, Alkalicarboxylate, wie z. B. Kaliumacetat, sowie Alkalimetallsalze von langkettigen Fettsäuren mit 10 bis 20 C-Atomen und gegebenenfalls seitenständigen OH-Gruppen. Bevorzugt als Alkalimetallsalz werden ein oder mehrere Alkalicarboxylate eingesetzt.

Als Katalysator **A4** kommen ferner in Betracht: Amidine, wie z. B. 2,3-Dimethyl-3,4,5,6-tetrahydropyrimidin, Tetraalkylammoniumhydroxide, wie z. B. Tetramethylammonium-hydroxid, Alkalihydroxide, wie z. B. Natriumhydroxid, und Tetraalkylammonium- oder Phosphoniumcarboxylate. Darüber hinaus sind Mannichbasen und Salze von Phenolen geeignete Katalysatoren.

Wird beim Verschäumen ein größerer Polyisocyanatüberschuss verwendet, kommen als Katalysatoren für die Trimerisierungsreaktion der überschüssigen NCO-Gruppen untereinander ferner in Betracht: Isocyanuratgruppen ausbildende Katalysatoren, beispielsweise Ammoniumionen- oder Alkalimetallsalze, speziell Ammonium- oder Alkalimetallcarboxylate, alleine oder in Kombination mit tertiären Aminen. Die Isocyanurat-Bildung führt zu besonders flammwidrigen PIR-Schaumstoffen.

Die oben genannten Katalysatoren können alleine oder in Kombination miteinander eingesetzt werden.

Gegebenenfalls können ein oder mehrere Hilfs- und Zusatzstoffe als Komponente **A5** eingesetzt werden. Beispiele für die Komponente **A5** sind oberflächenaktive Substanzen, Zellregler, Füllstoffe, Farbstoffe, Pigmente, Hydrolyseschutzmittel, fungistatische und bakteriostatisch wirkende Substanzen.

Als oberflächenaktive Substanzen kommen z.B. Verbindungen in Betracht, welche zur Unterstützung der Homogenisierung der Ausgangsstoffe dienen und gegebenenfalls auch geeignet sind, die Zellstruktur der Kunststoffe zu regulieren. Genannt seien beispielsweise Emulgatoren, wie die Natriumsalze von Ricinusölsulfaten oder von Fettsäuren sowie Salze von Fettsäuren mit Aminen, z.B. ölsaures Diethylamin, stearinsaures Diethanolamin, ricinolsaures Diethanolamin, Salze von Sulfonsäuren, z.B. Alkali- oder Ammoniumsalze von Dodecylbenzol- oder Dinaphthylmethandisulfonsäure und Ricinolsäure; Schaumstabilisatoren, wie Siloxanoxalkylen-Mischpolymerisate und andere Organopolysiloxane, oxethylierte Alkylphenole, oxethylierte Fettalkohole, Paraffinöle, Rizinusöl- bzw. Ricinolsäureester, Türkischrotöl und Erdnussöl, und Zellregler, wie Paraffine, Fettalkohole und Dimethylpolysiloxane.

Als Füllstoffe, insbesondere verstärkend wirkende Füllstoffe, sind die an sich bekannten, üblichen organischen und anorganischen Füllstoffe, Verstärkungsmittel, Beschwerungsmittel, Mittel zur Verbesserung des Abriebverhaltens in Anstrichfarben, Beschichtungsmittel usw. zu nennen. Im Einzelnen seien beispielhaft genannt: anorganische Füllstoffe wie silikatische Mineralien, beispielsweise Schichtsilikate wie z. B. Antigorit, Serpentin, Hornblenden, Amphibole, Chrisotil, Montmorillonit und Talkum, Metalloxide, wie Kaolin, Aluminiumoxide, Titanoxide und Eisenoxide, Metallsalze, wie Kreide, Schwerspat und anorganische Pigmente, wie Cadmiumsulfid und Zinksulfid, sowie Glas u.a. sowie natürliche und synthetische faserförmige Mineralien wie Wollastonit, Metall- und insbesondere Glasfasern verschiedener Länge, die gegebenenfalls geschlichtet sein können. Als organische Füllstoffe kommen beispielsweise in Betracht: Kohle, Melamin, Kolophonium, Cyclopentadienylharze und Pfropfpolymerisate sowie Cellulosefasern, Polyamid-, Polyacrylnitril-, Polyurethan-, Polyesterfasern auf der Grundlage von aromatischen und/oder aliphatischen Dicarbonsäureestern und Kohlenstofffasern.

Als geeignete organische Polyisocyanatkomponente **B** sind aliphatische, cycloaliphatische, araliphatische, aromatische und heterocyclische Polyisocyanate, wie sie z.B. von W. Siefken in Justus Liebigs Annalen der Chemie, 562, Seiten 75 bis 136 beschrieben werden, beispielsweise solche der Formel (IV),

$$Q(NCO)_n, \qquad (IV)$$

in der

n = 2 - 4, vorzugsweise 2 - 3,

und

Q einen aliphatischen Kohlenwasserstoffrest mit 2 - 18, vorzugsweise 6 - 10 C-Atomen, einen cycloaliphatischen Kohlenwasserstoffrest mit 4 - 15, vorzugsweise 6 - 13 C-Atomen oder einen araliphatischen Kohlenwasserstoffrest mit 8 - 15, vorzugsweise 8 - 13 C-Atomen bedeuten.

Beispielsweise handelt es sich um solche Polyisocyanate, wie sie in der EP 0 007 502 A1, Seiten 7 - 8, beschrieben werden. Bevorzugt werden in der Regel die technisch leicht zugänglichen Polyisocyanate, z.B. das 2,4- und 2,6-Toluylendiisocyanat, sowie beliebige Gemische dieser Isomeren ("TDI"); Polyphenylpolymethylenpolyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten. Die urethangruppenhaltigen Polyisocyanate (Präpolymere) können beispielsweise Reaktionsprodukte der Polyisocyanate mit Polyester-Polyolen oder aber beliebigen anderen Polyolen (beispielsweise konventionellen Polyetherpolyolen) sein. Vorzugsweise wird als Polyisocyanat mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt, besonders bevorzugt wird als Polyisocyanat eine Mischung enthaltend 4,4'-Diphenylmethandiisocyanat und 2,4'-Diphenylmethandiisocyanat und Polyphenylpolymethylenpolyisocyanat eingesetzt.

Es ist möglich, dass im Reaktionsgemisch die Anzahl der NCO-Gruppen im Isocyanat und die Anzahl der gegenüber Isocyanaten reaktiven Gruppen zu einer Kennzahl (Index) von 90 bis 600 führen, bevorzugt zwischen 115 und 400. Diese Kennzahl liegt bevorzugt in einem Bereich von 150 bis 450 in dem ein hoher Anteil an Polyisocyanuraten (PIR) vorliegt (der Hartschaumstoff wird als PIR-Hartschaumstoff oder PUR-/PIR-Hartschaumstoff bezeichnet) und zu einer höheren Flammwidrigkeit des PUR-/PIR-Hartschaumstoffes selbst führt. Ein anderer bevorzugter Bereich für die Isocyanat-Kennzahl ist der Wertebereich von 90 bis 150 (der Hartschaumstoff wird als Polyurethanhartschaumstoff (PUR-Hartschaumstoff) bezeichnet) mit zum Beispiel einer geringeren Sprödigkeit im Vergleich zu PUR-/PIR-Hartschaumstoff.

Unter der Isocyanat-Kennzahl (auch Kennzahl oder Isocyanat-Index genannt) wird der Quotient aus der tatsächlich eingesetzten Stoffmenge [Mol] an Isocyanat-Gruppen und der tatsächlich eingesetzten Stoffmenge [Mol] an Isocyanat-reaktiven Gruppen, multipliziert mit 100, verstanden:

$$\text{Kennzahl} = (\text{Mole Isocyanat-Gruppen} / \text{Mole Isocyanat-reaktive Gruppen}) * 100$$

Der NCO-Wert (auch: NCO-Gehalt, Isocyanatgehalt) wird bestimmt mittels EN ISO 11909 (Mai 2007).

Die Erfindung betrifft ebenfalls einen PUR-/PIR-Hartschaumstoff, der durch das erfindungsgemäße Verfahren hergestellt ist.

Die Herstellung der erfindungsgemäßen PUR-/PIR-Hartschaumstoffe erfolgt bevorzugt nach dem Fachmann bekannten Einstufenverfahren, bei dem die Reaktionskomponenten kontinuierlich oder diskontinuierlich miteinander zur Reaktion gebracht werden und dann anschließend entweder manuell oder durch Zuhilfenahme maschineller Einrichtungen im Hochdruck- oder Niederdruckverfahren nach Austrag auf ein Transportband oder in geeignete Formen zur Aushärtung gebracht werden. Beispiele sind in US-A 2 764 565, in G. Oertel (Hrsg.) "Kunststoff-Handbuch", Band VII, Carl Hanser Verlag, 3. Auflage, München 1993, S. 267 ff., sowie in K. Uhlig (Hrsg.) "Polyurethan Taschenbuch", Carl Hanser Verlag, 2. Auflage, Wien 2001, S. 83-102 beschrieben. Die PUR-/PIR-Hartschaumstoffe können eine Rohdichte von 25,0 bis 300,0 kg/$m^3$, bevorzugt 25,0 bis 80,0 kg/$m^3$, besonders bevorzugt 30,0 bis 65,0 kg/$m^3$ und insbesondere 30,0 bis 45,0 kg/$m^3$ aufweisen.

Die erfindungsgemäßen PUR-/PIR-Hartschaumstoffe finden bevorzugt zur Herstellung von Verbundelementen Verwendung. Üblicherweise findet hier die Verschäumung in kontinuierlicher oder diskontinuierlicher Weise gegen mindestens eine Deckschicht statt.

Ein weiterer Gegenstand der Erfindung ist demzufolge die Verwendung eines erfindungsgemäßen PUR-/PIR-Hartschaumstoffes als Isolationsschaum und/oder als Haftvermittler in Verbundelementen, wobei die Verbundelemente eine einen erfindungsgemäßen PUR-/PIR-Hartschaumstoff umfassende Schicht und mindestens eine Deckschicht umfassen. Die Deckschicht wird hierbei zumindest teilweise von einer den erfindungsgemäßen PUR-/PIR-Hartschaumstoff umfassenden Schicht kontaktiert. Verbundelemente der hier interessierenden Art werden auch als Sandwich-Elemente oder Dämmplatten bezeichnet und dienen in der Regel als Bauelemente für den Schallschutz, die thermische Dämmung, zum Hallenbau oder für den Fassadenbau. Es kann sich bei den einen oder zwei Deckschichten jeweils um eine flexible Deckschicht, z.B. um eine Aluminium-Folie, Papier, Kunststoffbahnen, Multischicht-Deckschichten aus Papier und Aluminium oder aus Mineralvließ, und/oder um eine starre Deckschicht, z.B. aus Stahlblech oder bis zu 7 mm starke Spanplatten, handeln, abhängig vom Einsatzzweck der Verbundelemente.

In einer ersten Ausführungsform betrifft die Erfindung somit ein Verfahren zur Herstellung von PUR-/PIR-Hartschaumstoffen, enthaltend

**A1** eine Isocyanatreaktive Komponente

**A2** Flammschutzmittel

**A3** Treibmittel

**A4** Katalysator

**A5** gegebenenfalls Hilfs- und Zusatzstoffe

**B** eine organische Polyisocyanatkomponente

dadurch gekennzeichnet, dass
die Komponente **A1** ein Tri-Urethan-Triol **A1.1** und eine Verbindung **A1.2** ausgewählt aus der Gruppe bestehend aus Polyetherpolyol, Polyesterpolyol, Polyethercarbonatpolyol und Polyetheresterpolyol enthält.

In einer zweiten Ausführungsform betrifft die Erfindung ein Verfahren gemäß der ersten Ausführungsform, dadurch gekennzeichnet, dass die Isocyanat-reaktive Komponente **A1** eine Verbindung **A1.2** mit einer Hydroxylzahl von 100 bis 550 mg KOH/g, bevorzugt 100 bis 450 mg KOH/g, besonders bevorzugt 100 bis 350 mg KOH/g enthält.

In einer dritten Ausführungsform betrifft die Erfindung ein Verfahren gemäß einer der Ausführungsformen 1 oder 2, dadurch gekennzeichnet, dass die Isocyanat-reaktive Komponente **A1** eine Verbindung **A1.2** mit einer OH-Funktionalität von 1,5 bis 4,0, bevorzugt 1,8 bis 3,0, besonders bevorzugt 1,9 bis 2,5 enthält.

In einer vierten Ausführungsform betrifft die Erfindung ein Verfahren gemäß einer der Ausführungsformen 1 bis 3, dadurch gekennzeichnet, dass die Verbindung **A1.2** ein Polyesterpolyol oder ein Polyetherpolyol, bevorzugt ein Polyesterpolyol ist.

In einer fünften Ausführungsform betrifft die Erfindung ein Verfahren gemäß einer der Ausführungsformen 1 bis 4, dadurch gekennzeichnet, dass die Verbindung **A1.2** ein Polyesterpolyol erhältlich aus der Reaktion aliphatischer und/oder aromatischer Dicarbonsäure mit mindestens einem aliphatischen Diol ist.

In einer sechsten Ausführungsform betrifft die Erfindung ein Verfahren gemäß einer der Ausführungsformen 1 bis 5, dadurch gekennzeichnet, dass das Tri-Urethan-Triol **A1.1** eine Struktur gemäß der Formel (II) aufweist,

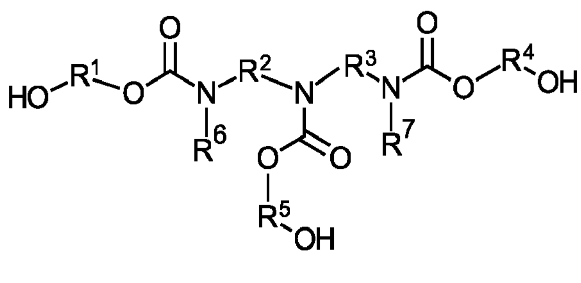

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^2$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können,

bevorzugt $C_2$ bis $C_{24}$ - Alkylen,

$R^3$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,

$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,

$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,

und wobei $R^1$ bis $R^7$ identisch oder voneinander verschieden sein können.

In einer siebten Ausführungsform betrifft die Erfindung ein Verfahren gemäß einer der Ausführungsformen 1 bis 6, dadurch gekennzeichnet, dass das Tri-Urethan-Triol **A1.1** erhalten wird aus der Reaktion zwischen cyclischen Ethylencarbonat und/oder cyclischen Propylencarbonat mit einer Verbindung enthaltend mindestens drei Aminogruppen, wobei mindestens zwei der Aminogruppen primäre Aminogruppen sind.

In einer achten Ausführungsform betrifft die Erfindung ein Verfahren gemäß einer der Ausführungsformen 1 bis 7, dadurch gekennzeichnet, dass der Anteil an Tri-Urethan-Triol **A1.1** 0,1 bis 35,0 Gew.-%, bevorzugt 3,0 bis 25,0 Gew.-%, besonders bevorzugt 5,0 bis 15,0 Gew.-%, bezogen auf die Summe der Massen der Komponenten **A1.1** und **A1.2**,beträgt.

In einer neunten Ausführungsform betrifft die Erfindung ein Verfahren gemäß einer der Ausführungsformen 1 bis 8, dadurch gekennzeichnet, dass die Isocyanat-Kennzahl 100 bis 500, bevorzugt 180 bis 450 beträgt.

In einer zehnten Ausführungsform betrifft die Erfindung ein Verfahren gemäß einer der Ausführungsformen 1 bis 9, dadurch gekennzeichnet, dass das Flammschutzmittel **A2** in einem Anteil von 2,0 Gew.-% bis 30,0 Gew.-%, bevorzugt 4,0 Gew.-% bis 25,0 Gew.-%, besonders bevorzugt 8,0 Gew.-% bis 20,0 Gew.-%, bezogen auf das Gewicht der Komponenten **A1** bis **A5.**

In einer elften Ausführungsform betrifft die Erfindung ein Verfahren gemäß einer der Ausführungsformen 1 bis 10, dadurch gekennzeichnet, dass als Polyisocyanatkomponente **B** mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt wird.

In einer zwölften Ausführungsform betrifft die Erfindung einen PUR-/PIR-Hartschaumstoff erhältlich durch ein Verfahren gemäß einer der Ausführungsformen 1 bis 11.

In einer dreizehnten Ausführungsform betrifft die Erfindung einen PUR-/PIR-Hartschaumstoff gemäß der zwölften Ausführungsform, dadurch gekennzeichnet, dass die Rohdichte 25,0 bis 300,0 $kg/m^3$, bevorzugt 25,0 bis 80,0 $kg/m^3$, besonders bevorzugt 30,0 bis 65,0 $kg/m^3$ und insbesondere 30,0 bis 45,0 $kg/m^3$ beträgt.

In einer vierzehnten Ausführungsform betrifft die Erfindung die Verwendung eines PUR-/PIR-Hartschaumstoffes gemäß einer der Ausführungsformen 13 oder 14 als Dämmmaterial oder Verbundelement mit flexiblen oder nicht-flexiblen Deckschichten.

In einer fünfzehnten Ausführungsform betrifft die Erfindung eine Mischung enthaltend ein Tri-Urethan-Triol **A1.1** und eine Komponente **A1.2,** dadurch gekennzeichnet, dass die Komponente **A1.2** ausgewählt ist aus der Gruppe bestehend aus Polyetherpolyol, Polyesterpolyol, Polyethercarbonatpolyol und Polyetheresterpolyol.

In einer sechszehnten Ausführungsform betrifft die Erfindung ein Verfahren gemäß einer der Ausführungsformen 1 bis 10, dadurch gekennzeichnet, dass das Tri-Urethan-Triol **A1.1** erhalten wird durch Umsetzung von cyclischen Carbonaten mit Triaminen gemäß Formel (III),

$$\mathbf{HN(R^6)\text{-}R^2\text{-}N(R^8)\text{-}R^3\text{-}NH(R^7)} \qquad \text{(III)}$$

wobei $R^2$, $R^3$, $R^6$, $R^7$ und $R^8$ die oben genannte Bedeutung haben, wobei $R^2$, $R^3$, $R^6$, $R^7$ und $R^8$ identisch oder voneinander verschieden sein können.

In einer siebzehnten Ausführungsform betrifft die Erfindung ein Verfahren gemäß einer der Ausführungsformen 1 bis 10, dadurch gekennzeichnet, dass das Tri-Urethan-Triol A1.1 erhalten wird aus der Reaktion zwischen cyclischem Propylencarbonat und/oder cyclischem Ethylencarbonat mit mindestens einer Verbindung ausgewählt aus der Gruppe bestehend aus Diethylentriamin, 1,5,10-Triazadecan, 1,12-Diamino-4,9-diazadodekan, Dipropylentriamin, N-(2-Aminoethyl)-1,3-propandiamin und N,N'-Bis(3-aminopropyl)ethylendiamin.

Die bevorzugten Ausführungsformen können einzeln oder auch miteinander verknüpft ausgeführt werden.

## Beispiele:

### Messmethoden:

| | |
|---|---|
| Hydroxylzahl: | Die Bestimmung der OH-Zahl erfolgte gemäß der Vorschrift der DIN 53240-1 (Verfahren ohne Katalysator, Fassung v. Juni 2013). |
| Aminzahl: | Gesamtbasenzahl gemäß DIN 51639-1 in der Fassung von Nov. 2014. |
| Säurezahl: | Die Säurezahl wurde gemäß DIN EN ISO 2114 (Juni 2002) bestimmt. |
| Abbindezeit: | Die Abbindezeit ("Gelpunkt $t_G$") wird ermittelt, indem man ein Holzstäbchen in die reagierende Polymerschmelze eintaucht und wieder herausnimmt. Sie charakterisiert den Zeitpunkt, ab dem sich die Polymerschmelze verhärtet. Als $t_G$ wird der Zeitpunkt angegeben, an dem sich erstmalig Fäden zwischen Holzstab und Polymerschmelze ziehen lassen. |
| Startzeit: | Die Zeitspanne, die vom Start der Vermischung der Hauptkomponenten bis zum sichtbaren Beginn der Schäumung des Gemisches verstreicht. |
| Viskosität: | Es wurde die dynamische Viskosität mit dem Rheometer MCR 51 der Firma Anton Paar entsprechend DIN 53019-1 (Fassung v. September 2008) mit einem Messkegel CP 50-1 (Durchmesser 50 mm, Winkel 1°) bei Scherraten von 25 $s^{-1}$, 100 $s^{-1}$, 200 $s^{-1}$ und 500 $s^{-1}$ gemessen. Die erfindungsgemäßen und nicht erfindungsgemäßen Polyole zeigen von der Scherrate unabhängige Viskositätswerte. |
| Rohdichte: | Die Rohdichte wurde gemäß DIN EN ISO 845 (Fassung vom Oktober 2009) ermittelt. |
| Brandeigenschaften: | Die Brandeigenschaften wurden gemäß DIN 4102-1 (Fassung v. Mai 1998) bestimmt. |
| Stauchhärte: | Die Stauchhärte der PUR-/PIR-Hartschaumstoffe wurden gemäß DIN EN ISO 844 (Fassung vom November 2014) bestimmt. |
| Offenzelligkeit: | Die Offenzelligkeit der PUR-/PIR-Hartschaumstoffe wurde nach DIN EN ISO 4590 (Fassung v. Juni 2014) ermittelt. |

### Eingesetzte Rohstoffe:

| | |
|---|---|
| cPC: | Cyclisches Propylencarbonat (Fa. Acros) |
| DBU: | 1,8-Diazabicyclo(5.4.0)undec-7-en |
| DETA: | Diethylentriamin |
| A1.2-1: | Aliphatisches Polyesterpolyol aus technischer Glutarsäure (Gemisch aus Glutarsäure, Bernsteinsäure und Adipinsäure, Fa. Lanxess AG) und Ethylenglykol, Hydroxylzahl: 237 mg KOH/g, Säurezahl: 1,9 mg KOH/g, Viskosität: 1160 mPas bei 25°C |
| A1.2-2: | Lineares Polyesterpolyol, Hydroxylzahl: 240 mg KOH/g (Desmophen®2382, Fa. Covestro Deutschland GmbH) |

A1.2-3: Enthaltend das Umsetzungsprodukt von Phthalsäureanhydrid und Diethylenglykol, Säurezahl: 97 mg KOH/g (Additiv 1132, Fa. Covestro Deutschland AG)
A1.2-4: Polyetherpolyol auf Aminbasis, Hydroxylzahl: 395 bis 435 mg KOH/g, Viskosität: 6450 - 9550 mPas bei 25°C (Desmophen®T 460, Fa. Covestro Deutschland GmbH)
A2-1: Trischlorisopropylphosphat (Levagard®PP, Fa. Lanxess AG)
A2-2: Triethylphosphat (Levagard®TEP-Z, Fa. Lanxess AG)
A3-1: n-Pentan (Fa. Kraemer&Martin GmbH)
A4-1: Aktivator für die Herstellung von Polyurethan-Hartschaumstoffen (Desmorapid®DB, Fa. Covestro Deutschland AG)
A4-2: Kaliumacetat in Diethylenglykol (Desmorapid®PU 1792, Fa. Covestro Deutschland AG)
A5-1: Modifiziertes Polyethersiloxan (Tegostab®B 8443, Fa. Evonik)
B-1: Polyisocyanat mit einem NCO-Gehalt von 30,5 bis 32,5 Gew.-% (Desmodur®44V20 L, Fa. Covestro Deutschland AG)

**Herstellung von Tri-Urethan-Triol A1.1-1:**

In einem 500 mL Planschliffreaktor mit beheizbarem Doppelmantel und elektrischem Rührer werden 348,7 g (3,42 mol) cPC und 2,22 g (14,6 mmol) DBU unter Inertgas ($N_2$) vorgelegt. Danach wird im Verlauf einer Stunde unter Rühren und 100 °C 50,3 g (0,49 mol) DETA zugetropft. Im Anschluss wurde die Reaktion für insgesamt 6 h bei 100 °C nachgerührt.
Überschüssiges cPC wurde durch Kurzwegverdampfung bei einem Druck von 0,01 mbar entfernt, wobei die Manteltemperatur 140 °C betrug und eine Dosiergeschwindigkeit von 200 g/Stunde gewählt wurde.

Analyse:

| | |
|---|---|
| Viskosität: | 95500 mPas (50 °C), 3900 mPas (75 °C) |
| Hydroxylzahl: | 366 mg KOH/g |
| Aminzahl: | 43,3 mg KOH/g |

**Herstellung einer Mischung enthaltend ein Tri-Urethan-Triol A1.1 und eine Verbindung A1.2:**

10 g des Tri-Urethan-Triols A1.1-2 werden in einem Trockenschrank auf 100°C vorgewärmt und zu 90 g auf 60°C vorgewärmtes A1.2-1 zugegeben. Die Mischung wird mittels eines Pendraulikrührers bis zur Homogenität verrührt und besitzt eine Hydroxylzahl von 250 mg KOH/g.

**Herstellung der PUR-/PIR-Hartschaumstoffe:**

Zur Herstellung der PUR-/PIR-Hartschaumstoffe werden die Isocyanatreaktiven Komponenten, Flammschutzmittel, Katalysatoren, Treibmittel und Schaumstabilisator vermischt, zu der erhaltene Mischung Polyisocyanat zugesetzt und das Gemisch in eine Papierform (30x30x10 $cm^3$) gegossen und darin ausreagiert. Die Rezepturen und Ergebnisse der physikalischen Messungen an den erhaltenen Proben sind in der Tabelle 1 wiedergegeben.

**Tabelle 1:** Herstellung von PUR-/PIR-Hartschaumstoffen unter Verwendung von A1.1-1

| **Beispiel** | | **1 (Vgl.)** | **2** | **3** | **4** |
|---|---|---|---|---|---|
| A1.2-2 | [g] | 74,9 | | | |
| Mischung enthaltend TUT A1.1-1 und Polyesterpolyol A1.2-1 | [g] | | 76,4 | 79,2 | 80,7 |
| A1.2-3 | [g] | 2,59 | 2,64 | 2,74 | 2,79 |
| A1.2-4 | [g] | 5,86 | 5,97 | 6,19 | 6,31 |
| A2-1 | [g] | 23,4 | 23,9 | 12,4 | 6,3 |
| A2-2 | [g] | 5,86 | 5,97 | 6,19 | 6,31 |
| A3-1 | [g] | 16,13 | 15,92 | 15,82 | 15,77 |
| A4-1 | [g] | 1,69 | 2,87 | 2,98 | 3,03 |

(fortgesetzt)

| Beispiel | | 1 (Vgl.) | 2 | 3 | 4 |
|---|---|---|---|---|---|
| A4-2 | [g] | 4,17 | 3,22 | 3,34 | 3,4 |
| A5-1 | [g] | 3,94 | 4,02 | 4,17 | 4,25 |
| B-1 | [g] | 211,4 | 209,1 | 217 | 221,1 |
| Isocyanat-Kennzahl | | 325,1 | 325,1 | 325,1 | 325,1 |
| Verarbeitung: | | | | | |
| Startzeit | [s] | 15 | 17 | 17 | 18 |
| Gelzeit | [s] | 43 | 47 | 46 | 46 |
| Klebfreizeit | [s] | 63 | 65 | 61 | 66 |
| Eigenschaften: | | | | | |
| Rohdichte, Kern | [kg/m$^3$] | 42,3 | 41,6 | 41,4 | 41,2 |
| Stauchhärte, quer | [kPa] | 225 | 202 | 205 | 209 |
| Stauchhärte, parallel | [kPa] | 375 | 318 | 336 | 342 |
| Offenzelligkeit | [%] | 5,2 | 5,7 | 5,7 | 5,1 |
| Flammhöhe | [mm] | 140-145 | 125-130 | 135-140 | 130-140 |
| Klassifizierung | | B2 | B2 | B2 | B2 |

Die Tabelle 1 zeigt in den Beispielen 2 bis 4, dass die Verwendung der erfindungsgemäßen Mischung zu PUR-/PIR-Hartschaumstoffen mit einer Brandschutzklasse B2 führt. Verglichen mit dem Vergleichsbeispiel 1 besitzen die PUR-/PIR-Hartschaumstoffe nach dem erfindungsgemäßen Verfahren (erfindungsgemäße Beispiele 2 bis 4) einen verbesserten Flammschutz (siehe Tabelle 1: Analyse). Die erfindungsgemäßen Beispiele 3 und 4 zeigen, dass der Anteil an Flammschutzmittel in den PUR-/PIR-Hartschaumstoffen, insbesondere des halogenhaltigen Flammschutzmittels TCPP, verringert werden kann. Der Flammschutz der PUR-/PIR-Hartschaumstoffen ist trotz eines verringerten Anteils an Flammschutzmittel mindestens gleichwertig zum Flammschutz der PUR-/PIR-Hartschaumstoffe des Vergleichsbeispiels 1.

Weiterhin belegt die Tabelle 1 (Analyse), dass alle verarbeitungstechnischen relevanten Größen, wie z.B. Startzeit, Gelzeit und Klebfreizeit gegenüber dem Vergleichsbeispiel 1 praktisch unverändert erhalten bleiben, wenn kleinere Anpassungen der Katalysatoren und Stabilisatoren erfolgen. Unter diesen Voraussetzungen behalten auch alle mechanischen Eigenschaften der PUR-/PIR-Hartschaumstoffe der erfindungsgemäßen Beispiele das vom Vergleichsbeispiel 1 vorgegebene Niveau bei.

## Patentansprüche

1. Verfahren zur Herstellung von PUR-/PIR-Hartschaumstoffen, enthaltend

    **A1** eine Isocyanatreaktive Komponente
    **A2** Flammschutzmittel
    **A3** Treibmittel
    **A4** Katalysator
    **A5** gegebenenfalls Hilfs- und Zusatzstoffe
    **B** eine organische Polyisocyanatkomponente

    **dadurch gekennzeichnet, dass**
    die Komponente **A1** ein Tri-Urethan-Triol **A1.1** und eine Verbindung **A1.2** ausgewählt aus der Gruppe bestehend aus Polyetherpolyol, Polyesterpolyol, Polyethercarbonatpolyol und Polyetheresterpolyol enthält.

2. Verfahren gemäß Anspruch 1, **dadurch gekennzeichnet, dass** die Isocyanat-reaktive Komponente **A1** eine Verbindung **A1.2** mit einer Hydroxylzahl von 100 bis 550 mg KOH/g, bevorzugt 100 bis 450 mg KOH/g, besonders

bevorzugt 100 bis 350 mg KOH/g enthält.

3. Verfahren gemäß Anspruch 1 oder 2, **dadurch gekennzeichnet, dass** die Isocyanat-reaktive Komponente **A1** eine Verbindung **A1.2** mit einer OH-Funktionalität von 1,5 bis 4,0, bevorzugt 1,8 bis 3,0, besonders bevorzugt 1,9 bis 2,5 enthält.

4. Verfahren gemäß einem der Ansprüche 1 bis 3, **dadurch gekennzeichnet, dass** die Verbindung **A1.2** ein Polyesterpolyol oder ein Polyetherpolyol, bevorzugt ein Polyesterpolyol ist.

5. Verfahren gemäß einem der Ansprüche 1 bis 4, **dadurch gekennzeichnet, dass** die Verbindung **A1.2** ein Polyesterpolyol erhältlich aus der Reaktion aliphatischer und/oder aromatischer Dicarbonsäure mit mindestens einem aliphatischen Diol ist.

6. Verfahren gemäß einem der Ansprüche 1 bis 5, **dadurch gekennzeichnet, dass** das Tri-Urethan-Triol **A1.1** eine Struktur gemäß der Formel (II) aufweist,

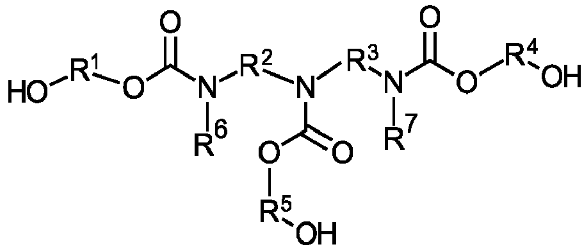

(II)

wobei

$R^1$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^2$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,
$R^3$ bedeutet lineares oder verzweigtes $C_2$ bis $C_{24}$ - Alkylen, $C_3$ bis $C_{24}$ - Cycloalkylen, $C_4$ bis $C_{24}$ - Arylen, $C_5$ bis $C_{24}$ - Aralkylen, $C_2$ bis $C_{24}$ - Alkenylen, $C_2$ bis $C_{24}$ - Alkinylen, die jeweils gegebenenfalls durch Heteroatome wie O, S oder N unterbrochen sein können und/oder jeweils mit Alkyl, Aryl und/oder Hydroxyl substituiert sein können, bevorzugt $C_2$ bis $C_{24}$ - Alkylen,
$R^4$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^5$ bedeutet lineares oder verzweigtes $C_2$ bis $C_{24}$ - Alkylen, das gegebenenfalls durch Heteroatome wie O, S oder N unterbrochen sein kann und substituiert sein kann, bevorzugt $CH_2$-$CH_2$ oder $CH_2$-$CH(CH_3)$,
$R^6$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,
$R^7$ bedeutet H, lineares oder verzweigtes $C_1$ bis $C_{24}$ - Alkyl, $C_3$ bis $C_{24}$ - Cycloalkyl, $C_4$ bis $C_{24}$ - Aryl, $C_5$ bis $C_{24}$ - Aralkyl, $C_2$ bis $C_{24}$ - Alkenyl, $C_2$ bis $C_{24}$ - Alkinyl, die jeweils gegebenenfalls durch Heteroatome wie O, S oder N unterbrochen sein können und/oder jeweils mit Alkyl, Aryl und/oder Hydroxyl substituiert sein können, bevorzugt H,
und wobei $R^1$ bis $R^7$ identisch oder voneinander verschieden sein können.

7. Verfahren gemäß einem der Ansprüche 1 bis 6, **dadurch gekennzeichnet, dass** das Tri-Urethan-Triol A1.1 erhalten wird aus der Reaktion zwischen cyclischen Ethylencarbonat und/oder cyclischen Propylencarbonat mit einer Verbindung enthaltend mindestens drei Aminogruppen, wobei mindestens zwei der Aminogruppen primäre Aminogruppen sind.

8. Verfahren gemäß einem der Ansprüche 1 bis 7, **dadurch gekennzeichnet, dass** der Anteil an Tri-Urethan-Triol

**A1.1** 0,1 bis 35,0 Gew.-%, bevorzugt 3,0 bis 25,0 Gew.-%, besonders bevorzugt 5,0 bis 15,0 Gew.-%, bezogen auf die Summe der Massen der Komponenten **A1.1** und **A1.2**, beträgt.

**9.** Verfahren gemäß einem der Ansprüche 1 bis 8, **dadurch gekennzeichnet, dass** die Isocyanat-Kennzahl 100 bis 500, bevorzugt 180 bis 450 beträgt.

**10.** Verfahren gemäß einem der Ansprüche 1 bis 9, **dadurch gekennzeichnet, dass** das Flammschutzmittel **A2** in einem Anteil von 2,0 Gew.-% bis 30,0 Gew.-%, bevorzugt 4,0 Gew.-% bis 25,0 Gew.-%, besonders bevorzugt 8,0 Gew.-% bis 20,0 Gew.-%, bezogen auf das Gewicht der Komponenten **A1** bis **A5.**

**11.** Verfahren gemäß einem der Ansprüche 1 bis 10, **dadurch gekennzeichnet, dass** als Polyisocyanatkomponente **B** mindestens eine Verbindung ausgewählt aus der Gruppe bestehend aus 2,4- und 2,6-Toluylendiisocyanat, 4,4'- und 2,4'- und 2,2'-Diphenylmethandiisocyanat und Polyphenylpolymethylenpolyisocyanat ("Mehrkern-MDI") eingesetzt wird.

**12.** PUR-/PIR-Hartschaumstoff erhältlich durch ein Verfahren gemäß einem der Ansprüche 1 bis 11.

**13.** PUR-/PIR-Hartschaumstoff gemäß Anspruch 12, **dadurch gekennzeichnet, dass** die Rohdichte 25,0 bis 300,0 $kg/m^3$, bevorzugt 25,0 bis 80,0 $kg/m^3$, besonders bevorzugt 30,0 bis 65,0 $kg/m^3$ und insbesondere 30,0 bis 45,0 $kg/m^3$ beträgt.

**14.** Verwendung eines PUR-/PIR-Hartschaumstoffes gemäß einem der Ansprüche 13 oder 14 als Dämmmaterial oder Verbundelement mit flexiblen oder nicht-flexiblen Deckschichten.

**15.** Mischung enthaltend ein Tri-Urethan-Triol **A1.1** und eine Verbindung **A1.2, dadurch gekennzeichnet, dass** die Verbindung **A1.2** ausgewählt ist aus der Gruppe bestehend aus Polyetherpolyol, Polyesterpolyol, Polyethercarbonatpolyol und Polyetheresterpolyol.

Europäisches Patentamt
European Patent Office
Office européen des brevets

# EUROPÄISCHER RECHERCHENBERICHT

Nummer der Anmeldung
EP 17 20 7933

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 571 167 A2 (BASF AG [DE]) 7. September 2005 (2005-09-07) * Beispiele 3,5 * | 1-5,8-15 | INV. C08G18/50 C08G18/66 C08G18/76 C08J9/14 C08G18/09 C08G18/18 C08G18/22 C08G18/38 C08G18/40 C08G18/42 C07C269/04 C07C271/20 |
| X | DE 27 31 012 A1 (MONTEDISON SPA) 12. Januar 1978 (1978-01-12) * Seite 4, Zeilen 1-5; Beispiele 1-6 * | 1-5,7-15 | |
| X | US 2006/160979 A1 (BENECKE HERMAN P [US] ET AL) 20. Juli 2006 (2006-07-20) * Beispiel C4 * | 15 | |
| X | WO 2015/138684 A2 (RUST OLEUM CORP [US]) 17. September 2015 (2015-09-17) * Beispiele 2,5-9,11 * | 15 | |
| A | WO 03/066580 A1 (UCB SA [BE]; VAN HOLEN JURGEN [BE]) 14. August 2003 (2003-08-14) * Seite 2, Zeilen 3,4; Beispiel 12 * | 1-15 | |
| A | EP 1 548 001 A1 (DAIKIN IND LTD [JP]) 29. Juni 2005 (2005-06-29) * Absätze [0001], [0051], [0052] * | 1-15 | RECHERCHIERTE SACHGEBIETE (IPC) |
| A | US 8 097 741 B2 (NDSU RES FOUNDATION) 17. Januar 2012 (2012-01-17) * Schema 1; Beispiel 1 * | 1-15 | C08G C08J C07C |
| A,D | EP 3 098 251 A1 (COVESTRO DEUTSCHLAND AG [DE]) 30. November 2016 (2016-11-30) * Anspruch 1 * | 1-15 | |

Der vorliegende Recherchenbericht wurde für alle Patentansprüche erstellt

1

| Recherchenort | Abschlußdatum der Recherche | Prüfer |
|---|---|---|
| München | 25. Mai 2018 | Lanz, Sandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 17 20 7933

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-05-2018

| Im Recherchenbericht angeführtes Patentdokument | Datum der Veröffentlichung | Mitglied(er) der Patentfamilie | Datum der Veröffentlichung |
|---|---|---|---|
| EP 1571167 A2 | 07-09-2005 | AT 402202 T | 15-08-2008 |
| | | DE 102004011346 A1 | 22-09-2005 |
| | | EP 1571167 A2 | 07-09-2005 |
| | | ES 2308319 T3 | 01-12-2008 |
| | | PT 1571167 E | 22-08-2008 |
| DE 2731012 A1 | 12-01-1978 | BE 856610 A | 09-01-1978 |
| | | CA 1114990 A | 22-12-1981 |
| | | CS 198260 B2 | 30-05-1980 |
| | | DE 2731012 A1 | 12-01-1978 |
| | | DK 301677 A | 10-01-1978 |
| | | ES 460546 A1 | 16-05-1978 |
| | | FI 772100 A | 10-01-1978 |
| | | FR 2357590 A1 | 03-02-1978 |
| | | GB 1589583 A | 13-05-1981 |
| | | IT 1064599 B | 18-02-1985 |
| | | JP S539741 A | 28-01-1978 |
| | | NL 7707427 A | 11-01-1978 |
| | | NO 772378 A | 10-01-1978 |
| | | SU 900816 A3 | 23-01-1982 |
| | | US 4120834 A | 17-10-1978 |
| US 2006160979 A1 | 20-07-2006 | KEINE | |
| WO 2015138684 A2 | 17-09-2015 | CA 2941109 A1 | 17-09-2015 |
| | | US 2016009852 A1 | 14-01-2016 |
| | | WO 2015138684 A2 | 17-09-2015 |
| WO 03066580 A1 | 14-08-2003 | AU 2003244476 A1 | 02-09-2003 |
| | | EP 1474383 A1 | 10-11-2004 |
| | | TW 200303301 A | 01-09-2003 |
| | | US 2005113594 A1 | 26-05-2005 |
| | | WO 03066580 A1 | 14-08-2003 |
| EP 1548001 A1 | 29-06-2005 | AU 2003252390 A1 | 23-02-2004 |
| | | CA 2493985 A1 | 12-02-2004 |
| | | CN 1675176 A | 28-09-2005 |
| | | EP 1548001 A1 | 29-06-2005 |
| | | JP WO2004013089 A1 | 21-09-2006 |
| | | US 2006091351 A1 | 04-05-2006 |
| | | WO 2004013089 A1 | 12-02-2004 |
| US 8097741 B2 | 17-01-2012 | US 2010216951 A1 | 26-08-2010 |
| | | WO 2008150568 A2 | 11-12-2008 |
| EP 3098251 A1 | 30-11-2016 | CN 107636042 A | 26-01-2018 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 17 20 7933

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-05-2018

| Im Recherchenbericht angeführtes Patentdokument | Datum der Veröffentlichung | Mitglied(er) der Patentfamilie | Datum der Veröffentlichung |
|---|---|---|---|
| | | EP 3098251 A1 | 30-11-2016 |
| | | WO 2016188838 A1 | 01-12-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3098251 A1
- EP 2910585 A1
- EP 1359177 A1
- WO 2010043624 A
- EP 1923417 A
- EP 0007502 A1
- US 2764565 A


### In der Beschreibung aufgeführte Nicht-Patentliteratur


- **B. INOUE et al.** Copolymerization of Carbon Dioxide and Epoxide with Organometallic Compounds. *Die Makromolekulare Chemie,* 1969, vol. 130, 210-220
- **IONESCU.** Chemistry and Technology of Polyols for Polyurethanes. *Rapra Technology Limited,* 2005, 55 ff
- **W. SIEFKEN.** *Justus Liebigs Annalen der Chemie,* vol. 562, 75-136
- Kunststoff-Handbuch. Carl Hanser Verlag, 1993, vol. VII, 267 ff
- Polyurethan Taschenbuch. Carl Hanser Verlag, 2001, 83-102